# EUROPEAN PATENT APPLICATION

(11) **EP 4 663 096 A1**
(43) Date of publication of application: **17.12.2025**
(21) Application number: 25174867.9
(22) Date of filing: 07.05.2025
(51) Int. Cl.: A61B 1/12, A61B 90/70

(54) **CONTAINING DEVICE FOR TREATING AND TRANSPORTING MEDICAL INSTRUMENTS IN A PROTECTED MANNER**

(30) Priority: 12.06.2024 IT 202400013420
(71) Applicant: STEELCO S.p.A., 31039 Riese Pio X (TV) (IT)
(72) Inventor: Pezzutti, Silvia, 31033 Castelfranco Veneto (TV) (IT); Pivotto, Stefano, 36027 Rosà (VI) (IT); Gambalonga, Leonardo, 35010 Massanzago (PD) (IT)
(74) Representative: Petraz, Gilberto Luigi

(57) **Abstract**

Containing device (10) for treating medical instruments in a protected manner, comprising a container (11) in which one or more medical instruments are put, at least one inlet (13) for a treatment liquid, and at least one lid (12) mobile with respect to the container (11) to selectively define a closed condition and an open condition.

## Description

### FIELD OF THE INVENTION

The present invention concerns a containing device for the protected treatment, in particular washing and sterilizing, and transport of medical instruments, for example flexible endoscopes, in a hospital or outpatient setting, both during their washing or treatment and also thereafter. The containing device according to the present invention can be used to treat and transport, in a protected manner, tubular type medical instruments made of various materials, in particular metal, plastic and/or rubber, used in medicine to explore, to examine by means of optical devices or to empty a body's natural cavities, and introduced through natural or artificial routes, such as for example rigid endoscopes, flexible fiberscopes, cystoscopes, ureteroscopes, resectors, light cables, glass syringes, endoscopic forceps and bridges, ring nuts, or other.

The present invention also concerns a method and a machine for treating medical instruments.

### BACKGROUND OF THE INVENTION

There are devices for the protected treatment, in particular washing and sterilizing, and transport of medical instruments in a hospital or outpatient setting, which provide a container in which the instruments to be treated are introduced and a lid that closes the container hermetically. These devices comprise, generally on the lid, an inlet for a treatment liquid, for example washing and sterilizing. These containers also provide outlets to discharge the liquid that has carried out a certain treatment.

An example of a protection device for washing endoscopic medical instruments is disclosed in international application WO2012/172403 in the name of the Applicant.

An example of a device for treating and transporting medical instruments in a protected manner is disclosed in international application WO2015/114578 in the name of the Applicant.

Machines for treating medical instruments, for example of the endoscopic type, used during clinical practice are known. These machines typically include a washing chamber, in which the complete washing treatment both of the external surfaces and also of the internal surfaces and channels of the endoscopic instrument is carried out, using washing liquids such as water and detergents, or chemical disinfectants, for example based on glutaraldehyde or peracetic acid.

The treatment of the external surface of the endoscopic instrument typically occurs by immersion in a treatment device as referred to above, positioned in the washing chamber and connected to the washing circuit by means of a sleeve. The treatment of the internal surfaces occurs by connecting flexible tubes to the internal channels of the endoscopic instrument.

There is a pressing need in the sector to guarantee that an endoscopic instrument, or medical instrument in general, keeps its sterility even after the treatment has taken place, and also during transport to the locations of use, or during storage at suitable warehouses awaiting transport to the locations of use.

A problem with these known treatment devices is that, in an operating situation inside the treatment machine, the treatment liquid does not remain inside the container for an amount of time that is optimal to carry out the operations for which it is intended, for example washing and sterilizing. The passage of the liquid inside the container can be, for example, too fast to guarantee an effective and homogeneous treatment of the medical instruments.

Known treatment devices of the type disclosed above therefore do not guarantee optimal treatment operations of the medical instruments, whether rigid or flexible, housed therein.

There is therefore the need to perfect a containing device for the protected treatment and transport of medical instruments that can overcome at least one of the disadvantages of the state of the art.

In particular, one purpose of the present invention is to provide a containing device for protected treatment and transport that guarantees effective treatment steps, for example washing and sterilizing, of both rigid as well as flexible medical instruments.

Another purpose of the present invention is to provide a containing device for the protected treatment and transport of medical instruments that allows to optimize the permanence of the treatment liquid inside the container in which they are housed.

Another purpose of the present invention is to perfect an effective and simple method for treating, for example washing and sterilizing, medical instruments.

Another purpose of the present invention is to provide a machine for treating medical instruments in which the containing device can be introduced.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a containing device for treating medical instruments in a protected manner comprises a container in which one or more medical instruments are put, at least one inlet for a treatment liquid and at least one lid mobile with respect to the container to selectively define a closed condition and an open condition of the containing device.

According to a characteristic aspect of the invention, the container comprises a bottom in which at least one hole is made, calibrated so as to allow a discharge of liquid with a flow rate lower than the flow rate loaded through the inlet.

Advantageously, the containing device guarantees effective treatment steps, such as washing and sterilizing, of both rigid as well as flexible medical instruments, and allows to optimize the permanence of the treatment liquid inside the container in which they are housed, thanks in particular to the presence on the bottom of the container of the discharge hole, which is suitably calibrated. This hole therefore substantially allows to slow down the exit of the liquid from the container and increase its permanence inside it, depending on the necessary times required for a specific type of treatment.

According to another aspect of the invention, the bottom internally has a concavity facing upward and the hole is positioned at the lowest point of the bottom.

According to another aspect of the invention, the hole is at least partly covered by a plate connected to the lid by means of attachment elements and configured to allow at least one slow flow of liquid from the hole, that is, a passage of liquid through a slot defined by the hole and the plate that partly covers it.

According to another aspect of the invention, one or more shims are positioned between the plate and the bottom.

According to another aspect of the invention, the lid comprises at least one hole for air to escape. This hole can be suitably calibrated and, thanks to the air exiting from the container, the formation of air bubbles and dead treatment zones is avoided or at least limited.

According to another aspect of the invention, the hole is at least partly covered by means of a plate connected to the lid by means of attachment elements and configured to allow at least one air vent from the hole.

According to another aspect of the invention, the lid internally has a concavity facing downward and the hole is positioned at the highest point of the lid.

According to another aspect of the invention, one or more shims are positioned between the plate and the lid.

The invention also concerns a method for treating medical instruments by means of the containing device as disclosed above, comprising positioning the medical instruments inside the container and sending treatment liquid inside the container through the at least one inlet, with a flow rate greater than the flow rate of liquid that exits from the at least one hole made on the bottom of the container.

The invention also concerns a machine for treating medical instruments, comprising a treatment chamber and at least one pump configured to send treatment liquid to at least one containing device as disclosed above, housed in the chamber.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a three-dimensional view of a containing device for treating and transporting medical instruments in a protected manner, in accordance with some embodiments described here;
- fig. 2 is a partly exploded top view of the containing device of fig. 1;
- fig. 3 is a partly exploded bottom view of the containing device of fig. 1;
- fig. 3A is an enlarged scale view of a detail of fig. 3;
- fig. 4 is a three-dimensional cross-section of the containing device of fig. 1;
- fig. 5 is a longitudinal section of the containing device of fig. 1;
- fig. 5A is an enlarged scale view of a detail of fig. 5;
- fig. 6 is a schematic representation of a treatment machine in which a containing device in accordance with some embodiments described here is positioned.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS

We will now refer in detail to the possible embodiments of the invention, of which one or more examples are shown in the attached drawings, by way of a non-limiting illustration. The phraseology and terminology used here is also for the purposes of providing non-limiting examples.

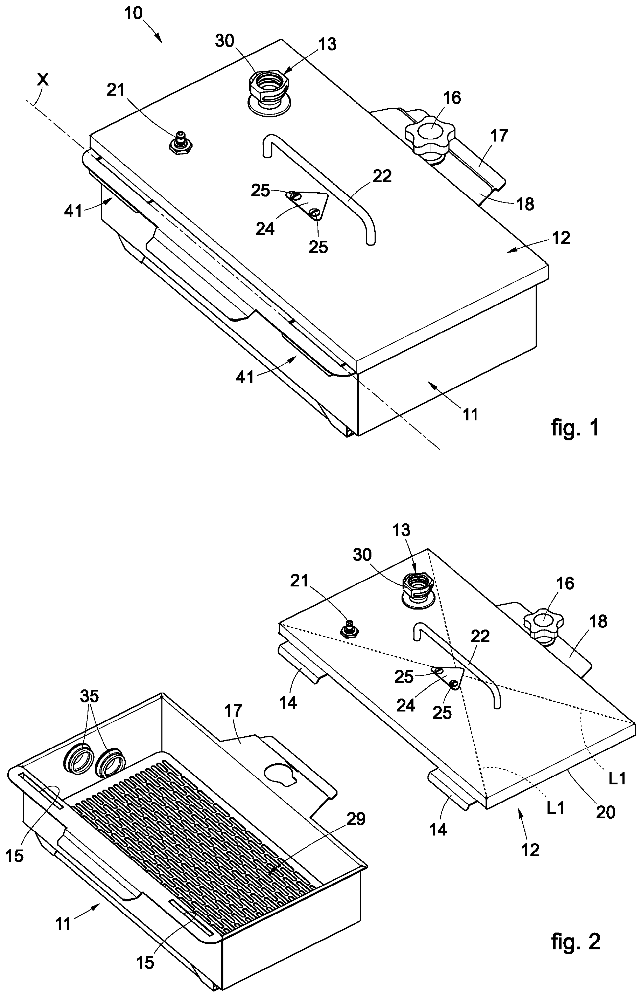

With reference to the attached drawings, see in particular fig. 1, fig. 2 and fig. 3, some embodiments are described of a containing device 10 for treating medical instruments in a protected manner, comprising a container 11 in which one or more medical instruments are put, an inlet 13 for a treatment liquid and a lid 12 mobile with respect to the container 11 to selectively define a closed condition and an open condition of the containing device 10. The container 11 can be made of plastic, for example by molding.

Depending on the treatment step, the liquid can be, for example, a washing, chemical sterilization, rinsing, or other liquid. The inlet 13 is made specifically on the lid 12.

The containing device 10 can be configured to be favorably portable.

In some embodiments, the lid 12 can be mobile with respect to the container 11 by being hinged, and thus rotatable, with respect to the container 11. In this case, the containing device 10 can be made in the shape of a small case or box with a hinged lid, in particular a portable case or box with a portable hinged lid. For example, the lid 12 and the container 11 can be considered the two complementary parts of a case.

Alternatively, the lid 12 can be mobile with respect to the container 11, being completely removable from the container 11. In this case, the containing device 10 can be made in the shape of a box with a completely removable lid, in particular a portable box.

In some example embodiments, the lid 12 can be, in particular, rotatable with respect to the container 11 around an axis X, along which hinges 41 are positioned, for example one, two, three or more than three. The hinges 41 are, for example, defined by suitably shaped hinging elements 14 made in the lid 12 or in the container 11, which are inserted in corresponding seatings 15 made on one side of the container 11 or lid 12.

Once closed, the lid 12 can be hermetically secured to the container 11 by means of a knob 16. The knob 16 allows to engage a protruding part 17 integral with the container 11 with a protruding part 18 integral with the lid 12. The protruding parts 17 and 18 are provided on the opposite side to the hinges 41.

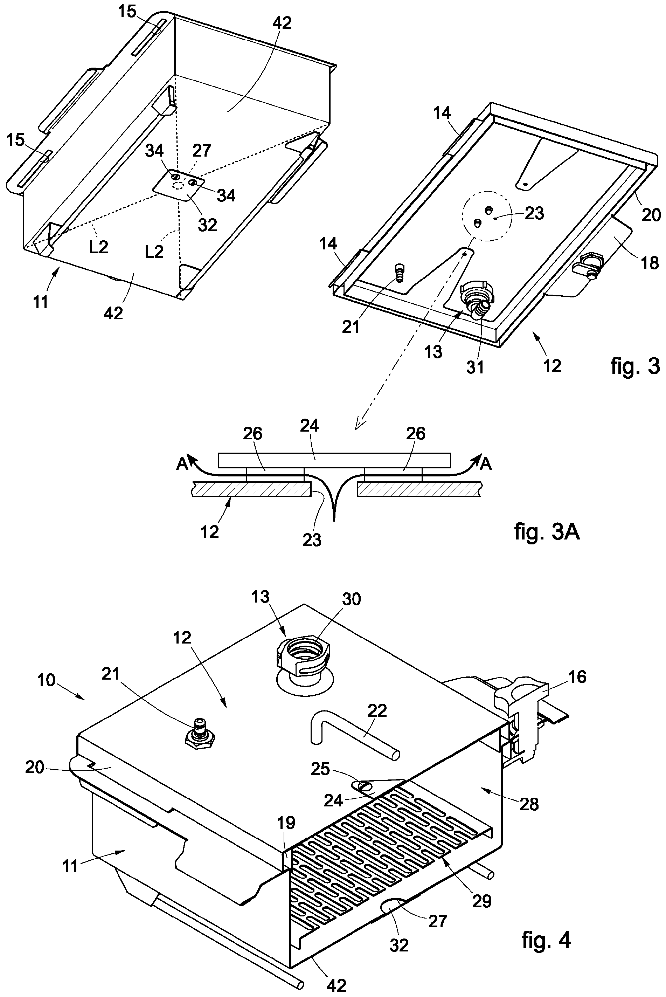

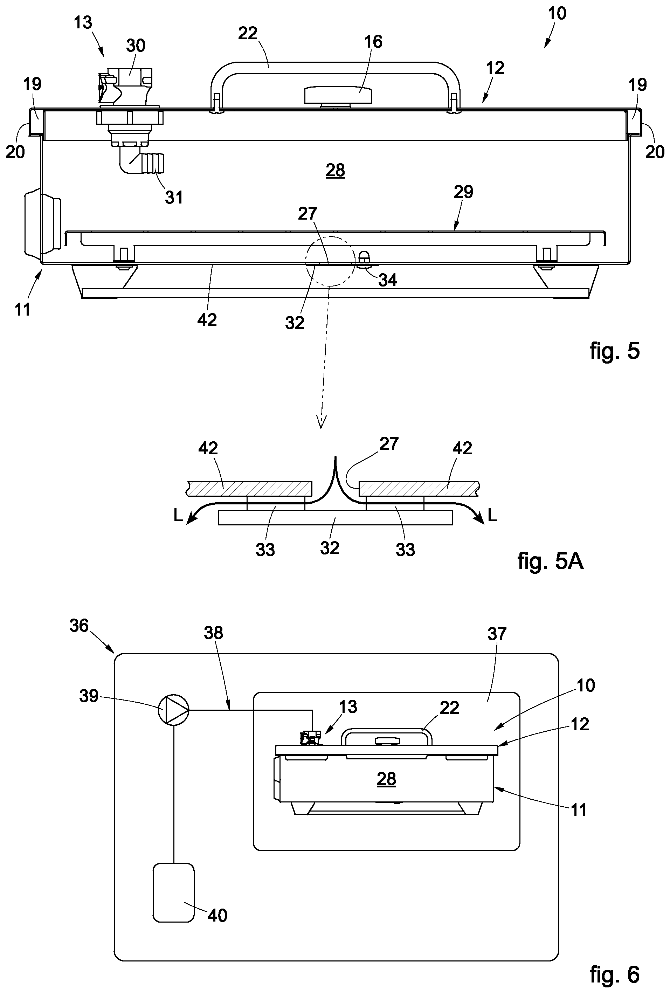

There is a hollow space 19 between the lid 12 and the container 11, see also fig. 4 and fig. 5, in which a sealing gasket can be positioned. In particular, the lid 12 is provided perimetrically with a lip 20 that partly overlaps with the sides of the container 11 when the lid 12 is closed.

The lid 12, in addition to the inlet 13, also comprises an attachment 21 to allow for what is known as a "leak test", in the event that the medical instrument to be treated is an endoscope or suchlike, comprising electrical parts and for which phenomena of liquid leakages into the electrical parts have to be avoided.

Externally, the lid 12 provides a transport handle 22 and, with reference to figs. 3-3A, it provides at least one vent hole 23 configured to allow air to escape from the upper part of the containing device 10. The hole 23 can be covered, specifically from the outside of the lid 12, by means of a plate 24, fig. 1 and fig. 2, which is connected to the lid 12 by means of attachment elements 25, such as screws or suchlike. The plate 24 is configured to allow at least one air A vent, even thin, from the hole 23. The plate 24 can be located at a certain distance from the hole 23 so that it can allow the passage of the air A, for example by means of shims 26, such as washers or suchlike, or other. This distance can also be adjusted by means of a greater or lesser tightening of the attachment elements 25. The plate 24 is positioned on the external surface of the lid 12.

The vent hole 23 therefore allows to remove the excess air from the containing device 10, avoiding or drastically limiting phenomena such as the formation of air bubbles in the treatment liquid during washing, sterilizing or rinsing operations. The hole 23 is always at least partly open and therefore allows for a constant discharge of air.

The lid 12 can have a downward-facing concavity and thus assume a dome, cone, pyramid or similar shape. The hole 23 is positioned at the apex, and therefore at the highest point, of the lid 12, so as to further facilitate the conveyance of the air toward the hole 23 and therefore outside of the containing device 10. The lid 12 therefore has a concavity facing downward, and therefore toward the container 11, when it is closed thereon. Purely by way of example, fig. 2 shows dashed lines L1 that represent a possible pyramid shape of the lid 12.

The container 11 comprises a chamber 28 into which the medical instruments to be treated are introduced. The medical instruments can be rested on a grid 29. The inlet 13 comprises a fitting 30 external to the lid 12 and a fitting 31 internal to the chamber 28. The fitting 31 can deliver treatment liquid directly into the chamber 28 or a further T-shaped fitting can be connected to it, which provides a connection branch, for example for an endoscopic instrument, and a branch that pours the liquid into the chamber 28.

The medical instruments to be treated in the chamber 28 can therefore be connected to the inlet 13 if they are, for example, endoscopic instruments or suchlike, and/or rested on the grid 29.

The container 11, see in particular figs. 4, 5, 5A, comprises a bottom 42 in which at least one hole 27 is made, calibrated to allow a discharge of liquid with a flow rate lower than the flow rate loaded through the inlet 13. In this way, the treatment liquid remains in the chamber 28 for a time sufficient to effectively carry out the operation for which it is intended, for example washing, sterilizing or rinsing. The hole 27 is substantially always open and allows for a controlled and calibrated discharge of liquid.

Internally, the bottom 42 can have an upward-facing concavity, therefore a concave, inverted pyramid or similar shape, and the hole 27 is positioned at the lowest point of the bottom 42, thus facilitating the conveyance of the liquid toward it. The grid 29 can be located in the chamber 28 at a certain distance with respect to the bottom 42. Fig. 3 shows dashed lines L2 that schematically represent an inverted pyramid shape of the bottom 42.

The hole 27 can be at least partly covered, in particular from the outside of the container 11, by means of a plate 32 connected to the bottom 42 by means of attachment elements 34, such as screws or suchlike. The plate 32 is configured to allow at least one slow flow of liquid L from the hole 27, that is, a passage of liquid through a slot defined by the hole 27 and the plate 32 that partly covers, as shown in fig. 5A. The plate 32 can be located at a certain distance from the hole 27 so that it can allow the discharge of the liquid L, for example by means of shims 33, such as washers or suchlike, fig. 5A, or other. This distance can also be adjusted by means of a greater or lesser tightening of the attachment elements 34. The plate 32 is positioned on the external surface of the bottom 42 and can be located at a minimum distance from the hole 27, such as to also allow a thin, slow flow of liquid L, for example if a longer permanence time of the liquid inside the container 11 is desired.

The container 11 can also provide one or more inspection windows 35 or portholes on one side. Alternatively, the windows 35 or portholes can be absent.

The containing device 10 is introduced into a machine 36 for treating, in particular washing, sterilizing and rinsing, medical instruments, fig. 6. The machine 36 comprises a treatment chamber 37 in which the containing device 10 is positioned.

The inlet 13 of the lid is connected, in particular by means of the fitting 30, to a hydraulic circuit 38 for distributing the treatment liquid. The treatment liquid is introduced into the containing device 10 by means of a pump 39 which draws it from at least one receptacle 40. By means of suitable control systems, it is possible to provide that the pump 39 receives, depending on the treatment step, a certain type of liquid coming from a corresponding container.

The pump 39 delivers treatment liquid into the chamber 28 of the containing device 10 with a flow rate greater than the one that exits the hole 27, which is calibrated for this purpose and is made on the bottom 42 of the container **11** of the containing device 10.

Heating means can also be provided inside the machine 36, configured to heat the liquid to be introduced inside the containing device 10.

A method for treating medical instruments therefore provides to position the medical instruments inside the container 11 and send treatment liquid inside the container 11 through the inlet with a flow rate greater than the flow rate of liquid that exits the hole 27 made on the bottom 42 of the container 11.

An example of a medical instruments chemical sterilization cycle inside the containing device 10, carried out by means of the machine 36, can provide the following steps:
- a step of initial discharge of liquid from the machine 36;
- loading a certain amount of treatment liquid into the containing device 10 and a certain amount into the machine 36;
- detergent dosage in percentage dependent on the type of washing to be carried out;
- heating the liquid to about 35°C;
- approximately 5 minutes permanence time of the medical instruments at 35°C inside the containing device 10;
- liquid discharge;
- new loading of liquid, partly in the containing device 10 and partly in the machine 36;
- disinfectant dosage in percentage dependent on the type of chemical sterilization to be carried out;
- heating the liquid to about 35°C;
- approximately 10 minutes permanence time of the medical instruments at 35°C inside the containing device 10;
- liquid discharge;
- new loading of liquid, partly in the containing device 10 and partly in the machine 36 without heating, for a rinsing operation;
- approximately 2 min. permanence time of the liquid in the device;
- liquid discharge;
- possible new loading of liquid, partly in the containing device 10 and partly in the machine 36 without heating, for an additional rinsing operation;
- approximately 2 min. permanence time of the liquid in the containing device 10;
- final liquid discharge;
- cycle ends.

It is clear that modifications and/or additions of parts may be made to the containing device for treating and transporting medical instruments in a protected manner as described heretofore, without thereby departing from the field and scope of the present invention, as defined by the claims.

In the following claims, the sole purpose of the references in brackets is to facilitate their reading and they must not be considered as restrictive factors with regard to the field of protection defined by the claims.

## Claims

1. Containing device (10) for treating medical instruments in a protected manner, comprising a container (11) in which one or more medical instruments are put, at least one inlet (13) for a treatment liquid, and at least one lid (12) mobile with respect to said container (11), to selectively define a closed condition and an open condition of said device (10), **characterized in that** said container (11) comprises a bottom (42) in which at least one hole (27) is made, calibrated so as to allow a discharge of liquid with a flow rate lower than the flow rate loaded through said inlet (13), said at least one hole (27) being at least partly covered by a plate (32), connected to said bottom (42) by means of attachment elements (34) and configured to allow at least a slow flow of liquid from said hole (27).

2. Containing device (10) as in claim 1, **characterized in that** said bottom (42) internally has a concavity facing upward and said hole (27) is positioned at the lowest point of said bottom (42).

3. Containing device (10) as in claim 1, **characterized in that** one or more shims (33) are positioned between said plate (32) and said bottom (42).

4. Containing device (10) as in any claim hereinbefore, **characterized in that** said lid (12) comprises at least one hole (23) for air to escape.

5. Containing device (10) as in claim 4, **characterized in that** said hole (23) is at least partly covered by a plate (24) connected to said lid (12) by means of attachment elements (25) and configured to allow at least one air vent from said hole (23).

6. Containing device (10) as in claim 4 or 5, **characterized in that** said lid (12) internally has a concavity facing downward and said hole (23) is positioned at the highest point of said lid (12).

7. Containing device (10) as in claim 5 or 6, **characterized in that** one or more shims (26) are positioned between said plate (24) and said lid (12).

8. Method for treating medical instruments by means of a containing device (10) as in any claim hereinbefore, **characterized in that** it comprises positioning said medical instruments inside said container (11) and sending treatment liquid inside said container (11) through said at least one inlet (13) with a flow rate greater than the flow rate of liquid that exits from the at least one hole (27) made on the bottom (42) of said container (11).

9. Machine for treating medical instruments, **characterized in that** it comprises a treatment chamber (37) and at least one pump (39) configured to send treatment liquid to at least one containing device (10) as in any previous claim from 1 to 7, housed in said chamber (37).
